# EUROPEAN PATENT APPLICATION

(11) **EP 2 042 234 A1**
(43) Date of publication of application: **01.04.2009**
(21) Application number: 08164941.0
(22) Date of filing: 23.09.2008
(51) Int. Cl.: B01J 20/10, G01N 33/49

(54) **Device for separation of particulates from a biological sample**

(30) Priority: 26.09.2007 US 861669
(71) Applicant: Honeywell International Inc., Morristown, NJ 07962 (US)
(72) Inventor: Gu, Yuandong, Plymouth, MN 55447 (US); Bardell, Ron L., Minneappolis, MN 55416 (US); Lukyanov, Anatoly, Seattle, WA 98133 (US)
(74) Representative: Buckley, Guy Julian

(57) **Abstract**

A device for separating plasma from blood cells involves a separation fiber having one or more characteristics such as a high silica content, a low boron content, and/or no binder.

## Description

### Field of the Invention

The disclosure relates generally to devices and methods for analyzing samples containing particles, and in particular, to a device for separating cells and other particulates from blood and other body fluids.

### Background

Detecting and/or measuring the concentration of blood and other body fluid components is a common tool used in hospitals, doctors' offices, and in the home to identify diseases and physical conditions. Biological fluids such as blood, urine or cerebrospinal fluids, which may at times contain blood, are frequently employed biological samples for such tests. Components often measured include metabolites, proteins, enzymes, antigens, antibodies, lipids, and electrolytes. These components are often measured using a plasma, serum, or cell-free sample obtained by centrifuging or filtering whole blood or other body fluid to separate out the cells and particulates. Centrifuging the sample is often unsuited for urgent, on-site, and field applications, and existing filtration devices often tend to clog or allow some cells to pass through.

### Summary

A device is provided for analyzing fluids involving separating cells and other particulates from a sample. The device includes a body and a separation region in the body. In one illustrative embodiment, the separation region contains a separation fiber that includes at least 99% non-crystalline silica. In some embodiments, the separation fiber has an average fiber diameter of from 0.75 µm to 1.59 µm, a density in the range of 0.048 g/cm³ to 0.096 g/cm³, and a boron content of less than 0.010%.

In another embodiment, a microfluidic cartridge is provided for analyzing particulate-containing body fluids. One illustrative cartridge may include a housing, a sample port in the housing, a separation region in fluid communication with the sample port, and an analysis chamber in fluid communication with the separation region. The separation region of the microfluidic cartridge may contain a separation fiber that includes at least 99% silica. In some embodiments, the separation fiber has an average fiber diameter of from 0.75 µm to 1.59 µm, a density in the range of 0.048 g/cm³ to 0.096 g/cm³, and a boron content of less than 0.010%.

A method of separating blood cells and particulates from whole blood is also provided. One illustrative method may involve the steps of providing a separation device including a separation fiber that includes at least 99% silica, and less than 0.010% boron content. In some embodiments, the separation fiber has an average fiber diameter of from 0.75 µm to 1.59 µm, and a density in the range of 0.048 g/cm³ to 0.096 g/cm³.

### Brief Description of the Drawings

FIG. 1 is a schematic diagram of a body fluid filtering unit according to the prior art, with labeled particles added;
FIG. 2 shows the movement of labeled particles through the prior art device of FIG. 1;
FIG. 3 is a schematic diagram of a body fluid filtering unit according to an illustrative embodiment of the invention, with labeled particles retained in the filtering material; and
FIG. 4 is a schematic diagram of a microfluidics cartridge according to an illustrative embodiment of the invention.

### Description

Separation of cells and other particulates from biological fluids such as plasma is often critical to the analysis of chemicals from both components. Lysing cells in the sample as a means of removing whole cells often introduces proteins and other interferents, and can complicate the subsequent analysis, especially analysis of plasma components.

Typically, bacteria range in size from about 1 µm to about 5 µm. Blood cells range in size from platelets, averaging about 1 µm to about 4 µm in diameter, to monocytes, ranging in size from about 10 µm to about 30 µm in diameter. In order to effectively filter out blood cells, bacterial and other particulates from a blood or body fluid sample, a material with improved particle retention properties over that achieved with glass wool is desired. In particular, a material having fibers with a diameter similar to or smaller than the diameter of the particles to be retained is desired.

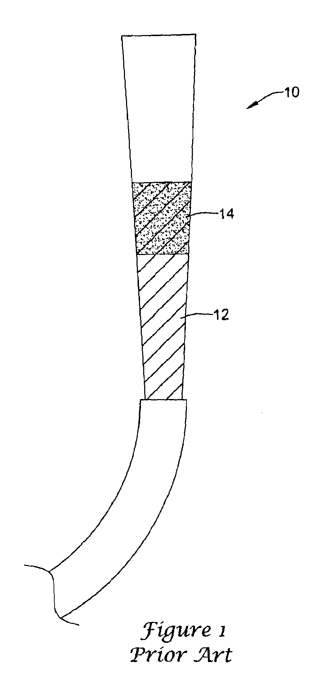

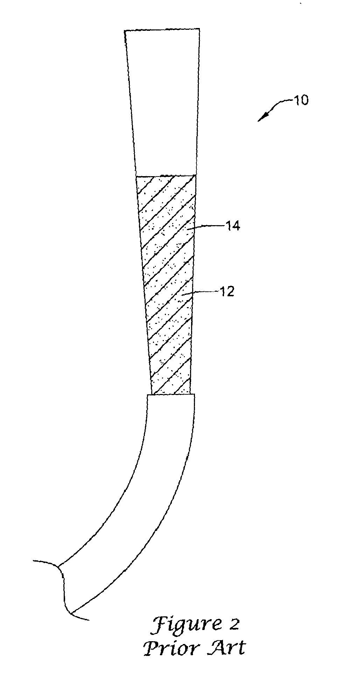

Prior art devices commonly used glass wool as a means of filtering blood cells from whole blood. FIGS. 1 and 2 illustrate the retention properties of glass wool in prior art devices. FIG. 1 shows a tube 10 packed with a layer of glass wool 12. To illustrate the filtration capabilities of the glass wool, and in one example, the tube is filled with phosphate buffered saline (PBS) by adding the buffer on top of the of glass wool layer 12. The tube 10 is connected to a peristaltic pump (not shown) and the pump is run at 200 µl/min until the PBS level reaches the upper level of glass wool. After that, 20 µl of carboxylate-modified yellow-green fluorescent microspheres 14 (1 µm diameter, available as FluoSpheres®, from Invitrogen, located in Carlsbad, California) are placed in the tube 10 containing glass wool 12. Empty space in the tube above the upper layer of glass wool 12 is filled with PBS. The pump is then run at 200 µl/min for 15 minutes, forcing PBS through the tube 10 in a downward direction. Removed PBS is replaced with fresh portions to keep the PBS level at least 3 mm above the upper layer of glass wool. FIG. 2 illustrates the fluorescent beads 14 moving through the glass wool 12 as the PBS is pumped through the tube 10. As can be seen, the 1 µm diameter beads 14 are not retained by the glass wool, but rather pass through the glass wool layer 12..

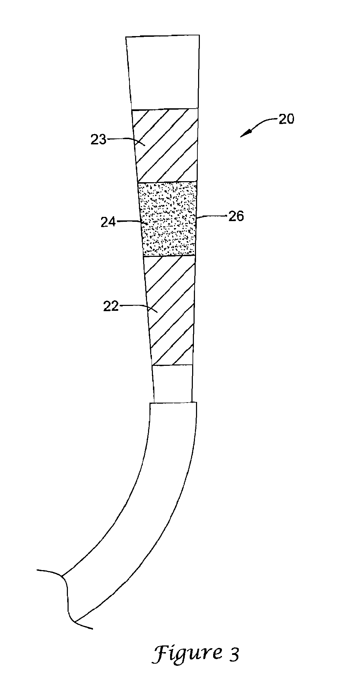

FIG. 3 illustrates the retention properties of an exemplary separation fiber of the present invention. It has been discovered that a silica fiber first developed for use in manufacturing tile sheathing for the U.S. Space Shuttle, sometimes referred to as Q-Fiber® (available from Johns Manville, Denver, CO), has good particle retention properties as compared to conventional borosilicate glass wool. Q-Fiber® has extremely high thermal capabilities and is ultra-light weight, which makes the fiber ideal for space shuttle applications. The silica fiber is very pure and stable, with little or no thermal expansion, contraction, or distortion.

Q-Fiber® was designed for applications requiring a fiber that does not degrade under extreme conditions. As a lightweight temperature-resistant insulation material that provides excellent sound absorption, Q-Fiber is used in a variety of aircraft and automotive uses.

Q-Fiber® has a low density, non-crystalline structure that provide superior insulation characteristics including a high degree of thermal resistance and stability, little or no degradation in extreme usage conditions, and is lightweight. Q-Fiber® forms the primary component for a diversity of insulating materials used in aerospace applications in which service temperatures range from -170°F (-112°C) to 2300°F (1260°C). Q-Fiber® is formed from high-silica-content sand which is melted, fiberized, acid-washed to remove impurities, rinsed, dried, and heat-treated for structural integrity. Q-Fiber® has a minimum silica content of about 99.7% after processing, and a boron content of less than 0.01%. Table 1 shows the typical chemical composition of Q-Fiber®.

**Table 1**

| Typical Chemical Composition of Q-Fiber® | |
|---|---|
| Oxide | Nominal Wt., % |
| SiO₂ | 99.9 |
| B₂O₃ | <0.01 |
| Fe₂O₃ | <0.01 |
| Al₂O₃ | <0.05 |
| CaO | <0.02 |
| MgO | <0.01 |
| Na₂O | <0.05 |

Q-Fiber® has an upper temperature limit of 2300°F (1260°C), and a continuous service temperature limit of 1800°F (982°C). The average fiber diameter ranges from 0.75 µm to 1.59 µm and examples of the nominal density of felted Q-Fiber® include 0.048 g/cm³, 0.056 g/cm³ and 0.064 g/cm³, and 0.096 g/cm³.

To illustrate the effectiveness of Q-Fiber®, and as shown in FIG. 3, a layer of conventional glass wool 22 is packed into tube 20. On top of the glass wool layer 22 is placed a small amount (approximately 0.1 cc) of Q-Fiber® 26. No binder, polymer, or other material is added to the Q-Fiber®. The Q-Fiber® is compressed. Another layer of glass wool 23 is added on top of Q-Fiber® layer 26. The tube 20 containing glass wool 22, 23 and Q-Fiber® 26 is connected to a peristaltic pump via a piece of tubing attached to the lower end of the tube. The tube is filled with PBS by adding the buffer to the top layer of glass wool 23. The pump is run at 200 µl/min until the PBS level reaches the upper level of glass wool 23. After that, 20 µl of carboxylate-modified yellow-green fluorescent microspheres 24 (1 µm diameter, available as FluoSpheres®, from Invitrogen, located in Carlsbad, California) are placed in the tube containing glass wool 23, 22 and Q-Fiber® 26. Empty space in the tube above the upper layer of glass wool 23 is filled with PBS. The pump is run at 200 µl/min for 15 minutes, forcing PBS through the tube. Removed PBS is replaced with fresh portions to keep the PBS level at least 3 mm above the upper layer 23 of glass wool. As shown in FIG. 3, the beads 24 pass through the top glass wool layer 23 but are quantitatively trapped by the Q-Fiber® 26 layer, and do not migrate into the lower glass wool layer 22.

In another experiment showing the separation of 1 µm diameter beads from a low molecular weight compound by Q-Fiber®, the experiment as described with reference to FIG. 3 was repeated with the beads dispersed in a 0.2 mg/ml solution of Rhodamine B (molecular weight 479.02, Merck Index: 13.8266). After pumping PBS through the tube for 10 minutes, Rhodamine B was completely removed while the beads remained trapped by Q-Fiber® layer 26.

In the illustrative embodiment, the binder-free Q-fiber^{®} 26 layer retains particles of 1 µm and larger, thus retaining bacterial cells, blood cells, and particulate components of blood, while permitting low molecular weight compounds to pass through.

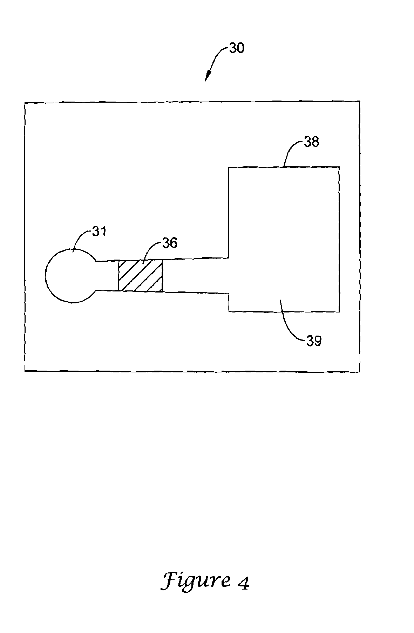

FIG. 4 is a schematic diagram of a microfluidics cartridge according to an illustrative embodiment. The cartridge includes a housing 30, a sample inlet port 31 in fluid communication with a separation fiber 36. The housing 30 may be made of any suitable material including metal, plastics, polymers, paper, and/or any other material suitable for small diagnostic devices. The microfluidics cartridge may be disposable. The separation fiber 36 is in fluid communication with an analysis chamber 38. In one illustrative embodiment, the separation fiber 36 may include at least 99% non-crystalline silica. In some embodiments, the separation fiber 36 has a boron content of less than 0.010% and no, or substantially no binder material. The separation fiber 36 may have a density in the range of 0.048 g/cm³ to 0.096 g/cm3. The separation fiber 36 may have an average fiber diameter of from 0.75 µm to 1.59 µm. The separation fiber 36 may be Q-fiber^{®}. In some embodiments, the analysis chamber 38 includes one or more reagents 39 used in the performance of an analysis Reagents 39 may also be contained in a reagent chamber (not shown) in fluid connection with the analysis chamber 38, if desired. During use, sample may be added to the inlet port 31. In some embodiments, one or more reagents may be added with the sample at the inlet port 31. The sample may travel through the separation fiber 36, where blood cells and other particulates are retained, and the cell-free portion of the sample may continue into the analysis chamber 38 via capillary, pumping, or other action.

Although the invention has been described with respect to at least one illustrative example, many variations and modifications will become apparent to those skilled in the art upon reading the present specification. It is therefore the intention that the appended claims be interpreted as broadly as possible in view of the prior art to include all such variations and modifications.

## Claims

**1.** original) A device for analyzing fluids, comprising:
a body (20);
a separation region in said body, said separation region containing a separation fiber (26), said separation fiber including at least 99% non-crystalline silica, wherein said separation region is substantially free of a binder.

**2.** original) The device of claim 1, wherein said separation fiber has an average fiber diameter of from 0.75 µm to 1.59 µm.

**3.** original) The device of claim 2, wherein said separation fiber has a density in the range of 0.048 g/cm³ to 0.096 g/cm³.

**4.** original) The device of claim 2, wherein said separation fiber has a boron content of less than 0.010%.

**7.** original) The device of claim 1, wherein said separation fiber is Q-fiber^{®}.

**8.** original) The device of claim 1, wherein the body includes a housing (30); the device further comprising a sample port (31) in the housing (30), wherein the separation region (36) is in fluid communication with the sample port (31); and an analysis chamber (38) in fluid communication with the separation region (36).

**9.** original) A method of separating cells and particulates from a body fluid sample, said method comprising the steps of:
providing a separation device including a separation fiber and an analysis zone in fluid communication with said separation fiber, said separation fiber including at least 99% silica and having less than 0.010% boron content, said separation fiber has an average fiber diameter of from 0.75 µm to 1.59 µm, said separation fiber has a density in the range of 0.048 g/cm³ to 0.096 g/cm³ wherein said separation fiber is substantially free of a binder;
providing a body fluid sample to said separation device; and
allowing said body fluid sample to contact said separation fiber.
said separation fiber has a density in the range of 0.048 g/cm³ to 0.096 g/cm³.

**10.** original) The method of claim 9, wherein said separation fiber is Q-fiber^{®}.
